(19) Europäisches Patentamt
European Patent Office
Office européen des brevets

(11) **EP 1 631 657 B1**

(12) **FASCICULE DE BREVET EUROPEEN**

(45) Date de publication et mention
de la délivrance du brevet:
**07.08.2013 Bulletin 2013/32**

(51) Int Cl.:
*C12G 1/02* *(2006.01)*   *C12H 1/00* *(2006.01)*
*C12N 1/20* *(2006.01)*

(21) Numéro de dépôt: **04767287.8**

(86) Numéro de dépôt international:
**PCT/FR2004/001421**

(22) Date de dépôt: **09.06.2004**

(87) Numéro de publication internationale:
**WO 2004/111179 (23.12.2004 Gazette 2004/52)**

(54) **SOUCHES MALOLACTIQUES TOLERANTES A L'ALCOOL POUR LA MATURATION DES VINS DE pH MOYEN OU ELEVE**

ALKOHOLTOLERANTE MALOLAKTISCHE STÄMME ZUR REIFUNG VON WEINEN MIT EINEM MITTLEREN ODER HOHEN PH-WERT

ALCOHOL-TOLERANT MALOLACTIC STRAINS FOR THE MATURATION OF WINES WITH AVERAGE OR HIGH PH

(84) Etats contractants désignés:
**AT BE BG CH CY CZ DE DK EE ES FI FR GB GR HU IE IT LI LU MC NL PL PT RO SE SI SK TR**

(30) Priorité: **12.06.2003 FR 0307046**

(43) Date de publication de la demande:
**08.03.2006 Bulletin 2006/10**

(73) Titulaire: **Danstar Ferment AG**
**6300 Zug (CH)**

(72) Inventeurs:
• **BOU, Magali**
**F-31600 Seysses (FR)**
• **KRIEGER, Sibylle**
**70469 Stuttgart (DE)**

(74) Mandataire: **Peaucelle, Chantal**
**Cabinet Armengaud Aîné**
**3, Avenue Bugeaud**
**75116 Paris (FR)**

(56) Documents cités:
WO-A-93/20180

• CARBO R ET AL: "AISLAMIENTO Y SELECION DE BACTERIAS LACTICAS EN VINO ISOLATION AND SELECTION OF LACTIC ACID BACTERIA IN WINE ISOLAMENTO E SELEZIONE DI BATTERI LATTICI NEL VINO" RIVISTA DI VITICOLTURA DI ENOLOGIA, SCARPIS, TREVISO,, IT, vol. 48, no. 4, 1995, pages 29-38, XP009021609 ISSN: 0370-7865

• CARRIE C ET AL: "COMPARISON OF COMMERICAL PREPARATIONS OF LACTIC ACID BACTERIA FOR DIRECT INOCULATION, FOR CONTROL OF MALOLACTIC FERMENTATION OF MERLOT WINES COMPARISON DE PREPARATIONS COMMERCIALES DE BACTERIES LACTIQUES A ENSEMENCEMENT DIRECT, EN VUE DE GERER LA FERMENTATION MALOLACTIQUE DU MERLOT" REVUE DES OENOLOGUES ET DES TECHNIQUES VITIVINICOLES ET OENOLOGIQUES, UNION NATIONALE DES OENOLOGUES FRANCE BOURGOGNE-PUBLICATIONS,, FR, no. 103, 2002, pages 16-18, XP009023946 ISSN: 0760-9868

• PILONE G J: "A NEW ZEALAND EXPERIENCE IN DIRECT-VAT INOCULATION FOR MALOLACTIC FERMENTATION" AUSTRALIAN AND NEW ZEALAND WINE INDUSTRY JOURNAL, AUSTRALIAN INDUSTRIAL PUBLISHERS, ADELAIDE, AU, vol. 10, no. 2, mai 1995 (1995-05), pages 169-173, XP009023865 ISSN: 0819-2421

• LIU S-Q ET AL: "GROWTH AND METABOLISM OF SELECTED LACTIC ACID BACTERIA IN SYNTHETIC WINE" AMERICAN JOURNAL OF ENOLOGY AND VITICULTURE, XX, XX, vol. 46, no. 2, 1995, pages 166-174, XP009021611 ISSN: 0002-9254

- JOYEUX A ET AL: "COMPARAISON DE DIVERSES PREPARATIONS INDUSTRIELLES DE BACTERIES LACTIQUES REACTIVEES POUR STIMULER LA FERMENTATION MALOLACTIQUE COMPARISON OF VARIOUS REACTIVATED INDUSTRIAL PREPARATIONS OF LACTIC ACID BACTERIA FOR STIMULATION OF MALOLACTIC FERMENTATION" CONNAISSANCE DE LA VIGNE ET DU VIN, VIGNE ET VIN PUBLICATIONS INTERNATIONALES, BORDEAUX, FR, vol. 19, no. 3, 1985, pages 149-159, XP009023986 ISSN: 0010-597X
- FUSTER A ET AL: "Improvement of the quality and typicalness of wines with the aid of new biological techniques." REVUE FRANCAISE D'OENOLOGIE, LALLEMAND SA, 130 ROUTE D'ESPAGNE, BP 1021, 31023 TOULOUSE, FRANCE, 2002, pages 28-31, XP009027063
- EDWARDS C G ET AL: "OCCURRENCE AND CHARACTERIZATION OF LACTIC ACID BACTERIA FROM WASHINGTON STATE WINES: PEDIOCOCCUS SPP" AMERICAN JOURNAL OF ENOLOGY AND VITICULTURE, XX, XX, vol. 43, no. 3, 1992, pages 233-238, XP009021610 ISSN: 0002-9254
- LIU S Q: "Malolactic fermentation in wine - beyond deacidification." JOURNAL OF APPLIED MICROBIOLOGY 92 (4) 589-601, NEW ZEALAND DAIRY RES. INST., PALMERSTON NORTH, NEW ZEALAND. E-MAIL SHAO.LIU(A)NZDRI.ORG.NZ, 2002, XP002272274

**Description**

La présente invention concerne un procédé de contrôle de la fermentation malolactique dans les vins par inoculation directe de souches de bactéries lactiques sélectionnées.

Elle a pour objet des souches de bactéries lactiques sélectionnées résistantes à l'alcool, capables d'initier et de réaliser une fermentation malolactique (FML) complète lorsqu'elles sont introduites sans étape préalable d'acclimatation dans un vin de pH moyen à élevé. Cette résistance à l'alcool est mise à profit dans un procédé de conduite de la FML dans un vin de pH moyen à élevé, également objet de la présente invention.

La fermentation malolactique (FML) est la décarboxylation de acide malique en acide lactique, résultant de l'activité métabolique de certaines bactéries lactiques. Issues de la surface des grains de raisin, des feuilles de vigne, du sol et du matériel vinicole, de nombreuses bactéries lactiques sont naturellement présentes à toutes les étapes du procédé de5.9 vinification et du stockage du vin. Toutefois, seuls quelques groupes de bactéries lactiques sont capables de se multiplier dans le jus de raisin et surtout dans le vin, en raison des conditions de croissances limitantes. Elles appartiennent à 4 genres: Oenococcus, Leuconostoc, Lactobacillus et Pediococcus. Trois espèces de Pediococcus et sept espèces de Lactobacillus se rencontrent communément dans le vin (Kunkee,1967, Adv. Appl. Microbiol., vol 9, pp. 235-279), tandis que Leuconostoc et Oenococcus y sont représentées chacune par une seule espèce (*Leuconostoc mesenteroides* et *Oenococcus oeni,* respecti ement).

Il est fait référence à la FML dans des articles scientifiques: Carbo et al., Riv. Vitic. Envl, vol. 48, No. 4., 1995, p29. 38; Carrie et al., Revue des Oenoloques, No 103, 2002, p. 16- 18; Pilone, Wine Industrie Journal, vol. 10, no. 2, 1995, p.169-173; Lin et al., Am. J. Envl. Vitric, vol 46, No. 2, 1995, p. 166-174; Joyeux et al., Connaissance Vigne Vin, vol. 19, No. 3, 1985, p. 149-159.

La diminution de l'acidité et la modification des arômes du vin qui découlent de la FML sont considérées comme bénéfiques à la qualité du vin. Cette fermentation secondaire contribue en outre à la stabilisation biologique du vin. Sa bonne réalisation revêt donc une grande importance pour l'obtention de vins de qualité, ce qui a amené les producteurs à rechercher des moyens de contrôler son initiation et son déroulement

Dans la vinification traditionnelle, la FML se produit grâce à la croissance spontanée d'une flore indigène de bactéries lactiques. Le processus de FML se déclenche de lui-même, lorsque la flore malolactique est suffisamment développée, c'est-à-dire de manière aléatoire entre la fin de fermentation alcoolique et plusieurs semaines, voire plusieurs mois, après la fermentation alcoolique. Lorsque les bactéries malolactiques atteignent une concentration d'environ 106 UFC/ml dans le milieu, elles entrent en phase métabolique active et démarrent la fermentation de l'acide malique. Dans ces conditions, *Oenococcus oeni* est l'espèce la plus fréquemment responsable de la FML. En effet, si en début de fermentation alcoolique, une prédominance des espèces homofermentaires *Lactobacillus plantarum* et *Lactobacillus casei* est observée, celles-ci disparaissent quand la teneur en alcool augmente. Après fermentation alcoolique ce sont les espèces Pediococcus et Oenococcus, selon le pH, qui prédominent et atteignent finalement la concentration critique pour déclencher la FML.

Plusieurs méthodes destinées à mieux contrôler le déclenchement et le déroulement de la FML ont été proposées, reposant principalement sur l'induction de la FML par inoculation des moûts ou des vins avec des souches de bactéries lactiques sélectionnées. En général, sont utilisées des souches *d'Oenococcus oeni,* cette espèce étant connue pour convenir le mieux à la FML.

Un premier procédé consiste à induire la FML à l'aide d'une biomasse non proliférante *d'Oenococcus oeni.* Du fait que les bactéries agissent dans ce cas comme une préparation enzymatique, la dégradation complète de l'acide malique n'est obtenue qu'avec une inoculation massive, à raison d'au moins $10^8$ UFC/ml, ce qui représente un coût bien trop élevé pour trouver une application pratique réelle.

Un deuxième procédé consiste à introduire dans la cuve de vinification des préparations *d'Oenococcus oeni,* avant le démarrage de la fermentation alcoolique, quand aucun alcool n'est encore produit et que le moût est riche en nutriments. Cette méthode a pour avantage que les préparations peuvent être ajoutées directement au moût à l'état lyophilisé ou congelé, dans la mesure où elles n'ont pas à subir un stress alcoolique qui leur serait fortement préjudiciable. Mais, les résultats ne sont pas satisfaisants, notamment en raison de la compétition entre levures et bactéries lactiques: une des deux populations se multiplie plus rapidement et peut supplanter l'autre, notamment sous l'influence du pH du milieu. En outre, *Oenococcus oeni* étant hétérofermentaire, elle peut utiliser le sucre comme substrat pour produire de l'acide acétique. De ce fait, le risque de voir une production d'acidité volatile par les bactéries lactiques est fortement dissuasif pour les viticulteurs (Riberau-Gayon et al., 1975, Sciences et techniques du vin, Tome III, Dunod, Paris).

Face à ce problème, il a été fait appel à des souches homofermentaires de *Lactobacillus plantarum,* de manière à ce qu'il n'y ait pas de production d'acide acétique à partir des sucres présents dans le moût ou dans le jus de fruits, et donc pas d'augmentation de la l'acidité volatile du vin (demande de brevet EP 0 398 957). Les souches sélectionnées sont introduites dans le moût ou le jus de fruit, avant ou dans les premiers moments de la FA. Cependant, ces bactéries malolactiques étant incapables de survivre dans un vin fermenté, la dégradation de l'acide malique ralentit et s'arrête quand le niveau d'alcool atteint 5%. Finalement, l'acide malique n'est pas totalement dégradé et le vin obtenu n'est pas bactériologiquement stable.

Une autre solution pour éviter la production d'acidité volatile à partir des sucres peut consister à ensemencer le vin seulement après la fermentation alcoolique, quand le taux de sucre est minimum. Les préparations de bactéries lactiques inoculées dans les cuves de vinification doivent dans ce cas être capables de survivre malgré le stress dû à un degré d'alcool déjà élevé dans le milieu, les bactéries lactiques étant d'autant plus sensibles au stress qu'elles sont introduites dans le milieu à l'état lyophilisé ou congelé.

Des préparations d'*Oenococcus oeni* sont commercialisées depuis plusieurs années pour l'ensemencement du vin après fermentation alcoolique. Une pré-culture dans un milieu enrichi en vin est préconisée, pour éviter la chute importante de la population cellulaire lors de l'inoculation en milieu alcoolisé. Par exemple, une incubation à 25°C durant 1 à 6 jours, permet d'améliorer la survie des bactéries en les plaçant dans un état physiologique adapté. Cette "réactivation", aussi appelée "acclimatation", doit permettre une induction des mécanismes de résistance des cellules confrontées à une teneur en alcool élevée, autorisant une augmentation de la population bactérienne et une reprise de l'activité métabolique (Lafon-Fourcade et al, 1993, Conn. Vigne Vin, vol 17, pp. 55-71). Cette méthode bien qu'efficace, demande du temps, du travail et requiert certaines connaissances microbiologiques. Le temps de pré-culture et le moment de l'introduction doivent être respectés précisément sous peine d'une perte significative de viabilité, ce qui représente un contrainte sérieuse pendant la période des vendanges.

Plus récemment, une autre voie a été explorée, pour disposer de préparations de bactéries lactiques répondant à la-double exigence de pouvoir être inoculées dans un vin sous forme lyophilisée ou congelée, sans perte de viabilité malgré le degré alcoolique, ce qui permet en particulier de réaliser la FML dans des délais plus courts sans augmenter la biomasse apportée. Le brevet EP 635 050 décrit un procédé d'induction de la fermentation malolactique par inoculation d'un vin avec une culture lyophilisée de bactéries lactiques appartenant au genre *Oenococcus oeni* résistantes à l'alcool, sans étape préalable d'acclimatation. Les souches *d'Oenococcus oeni* sélectionnées présentent un taux de survie élevé qui rend possible le déclenchement de la FML même à des basses concentrations d'apport ($1.10^6$ à $5.10^7$ CFU/ml) et en présence d'alcool à des taux compris entre 10,5% et 13%. Dans les conditions définies et pour un pH compris entre 3,2 et 3,6, les cellules entrent rapidement en phase active de fermentation malolactique.

Bien que ces préparations lyophilisées d'*Oenococcus oeni* pour l'ensemencement direct aient prouvé leur utilité dans divers types de vins, il existe de nombreux cas dans lesquels leur utilisation n'est pas satisfaisante. En effet, le pH du vin est un facteur essentiel qui intervient dans la sélection de l'espèce qui réalisera la FML.

Or une grande partie des vins rouges actuellement produits proviennent des régions chaudes (comme par exemple le sud de l'Europe). Ces vins ont un pH assez élevé, c'est-à-dire de l'ordre de 3,5 ou supérieur à cette valeur, ce qui favorise la croissance bactérienne et en premier lieu le développement des bactéries indigènes, malolactiques ou autres. Ces bactéries, qui résistent mal aux pH inférieurs à 3,5, sont alors favorisées au détriment d'*OE. oeni.*

Ceci conduit à deux types de problèmes. D'une part, bien que les starters de FML à base d'*Oe, oeni* qui sont inoculés dans les cuves après la fermentation alcoolique soient résistants à l'alcool, leur réhydratation et leur acclimatation demande un certain temps avant la reprise d'une activité fermentative. Or la microflore indigène étant favorisée aux pH élevés, des espèces indésirables peuvent se développer plus rapidement et supplanter *Oe. oeni.* La population bactérienne inoculée régresse au-dessous de la masse critique de 106 UFC/ml, voire ne l'atteint jamais. La dégradation de l'acide malique peut ne pas être complètement réalisée ou même ne pas être initiée du tout

D'autre part, aux pH favorisant la croissance bactérienne, on assiste fréquemment au déclenchement spontané de la FML par une ou plusieurs souches malolactiques indigènes, avant même que la fermentation alcoolique soit terminée, à un stade du processus où les sucres sont encore présents en quantité notable, dissuadant d'introduire des bactéries lactiques hétérofermentaires dans le milieu.

Dans les deux cas, le contrôle de la FML n'est simplement pas possible, puisque le risque est important qu'elle soit réalisée par des bactéries indéfinies, dont on ne connaît pas les caractéristiques. Les espèces indigènes dominantes peuvent en particulier consommer plusieurs substrats et produire des composés toxiques ou conduisant à des modifications organoleptiques désagréables et d'autres effets négatifs sur le vin.

On peut citer à titre d'exemple, certaines souches de *Lactobacillus brevis,* capables de métaboliser l'acide tartrique (maladie de la tourne), ce qui réduit l'acidité totale et augmente l'acidité volatile. Certaines souches de *Lactobacillus brevis* et *Lactobacillus buchneri* sont capables de dégrader le glycérol, les métabolites produits jouant alors le rôle de précurseur de l'acroléïne, qui réagit avec les tannins des vins et donne un goût amer. De nombreuses souches malolactiques, de Pediococcus, mais aussi de *Lactobacillus brevis et* certaines souches *d'Oenococcus oeni* peuvent décarboxyler les acides aminés en amines biogènes, comme l'histidine en histamine, qui peuvent être à l'origine de sévères réactions allergiques chez les personnes sensibles. *Leuconostoc mesenteroides, Lactobacillus brevis* et d'autres espèces, notamment des Pédiocoques sont responsables des vins filants, maladie des vins causée par la production de polysaccharides exocellulaires qui augmentent la viscosité du vin.

La présence de telles bactéries lactiques au sein d'une microflore indigène est un phénomène largement répandu. La complexité de cette microflore renforce les risques qu'au moins une souche indésirable soit présente dans le moût, et les valeurs de pH moyennes ou élevées lui donnent l'opportunité de s'y développer rapidement. Toutes ces réactions métaboliques participent d'une façon ou d'une autre à l'altération de la qualité du vin et sont à l'origine de pertes économiques sérieuses.

Les producteurs, qui ont des objectifs quantitatifs, mais aussi qualitatifs par rapport aux produits afin de répondre à l'attente du marché, doivent pouvoir maîtriser le plus possible le procédé de vinification quel que soit le pH du vin, et limiter les risques d'évolution non désirée tout au long du processus. Ils souhaitent disposer de starters de la FML adaptés aux conditions modernes de productions et aux exigences des consommateurs, qui les garantissent au mieux de ces risques.

La présente invention a pour but de répondre à ce besoin. De manière inattendue, la solution réside dans la sélection de souches de bactéries lactiques résistantes à l'alcool appartenant aux espèces Lactobacillus et Pediococcus. Elles sont capables d'initier et de réaliser une FML complète lorsqu'elles sont introduites sans étape préalable d'acclimatation dans un vin de pH moyen à élevé. Cette résistance à l'alcool, inédite chez les bactéries lactiques de ces espèces, se manifeste d'une part par un excellent taux de survie lors de l'inoculation et d'autre part par un démarrage rapide de l'activité fermentative.

Ces souches, outre qu'elles sont tolérantes à l'alcool, présentent un métabolisme homofermentaire, leur permettant d'être inoculées dans le vin avant la fin de la fermentation alcoolique, sans produire d'acidité volatile.

Les caractéristiques combinées de résistance à l'alcool et de croissance rapide à pH moyen ou élevé confèrent aux souches selon l'invention l'avantage essentiel de se développer plus rapidement que la flore indigène, même aux pH favorisant la croissance de cette dernière. Le résultat particulièrement avantageux en est que les espèces indigènes ne peuvent pas se développer, et sont ainsi empêchées de produire des composés indésirables. Un ensemencement en cours de fermentation alcoolique est possible sans nuire à la qualité du vin, ce qui offre une garantie supplémentaire que la FML soit réalisée sous le contrôle permanent du viticulteur.

Ce résultat est d'autant plus surprenant que les souches sélectionnées répondant aux exigences ci-dessus exposées appartiennent aux espèces Lactobacillus et Pediococcus. Ces bactéries lactiques ont jusqu'à présent été écartées d'un usage en milieu alcoolique, en particulier du fait qu'elles sont rarement présentes dans les milieux de fermentation alcoolisés et à plus forte raison dans les vins. Les rares tentatives de renverser cet a priori ont conduit à des résultats décevants.

Par exemple, il a été vu plus haut que l'ensemencement avec des préparations de *Lactobacillus plantarum* avant la fermentation alcoolique ne permettait pas une FML complète en raison de la perte d'activité quand le degré d'alcool atteint 5%. Plus récemment, cet état de fait a été confirmé par les travaux menés à l'Université de Rioja en Espagne (Tenorio *et al.,* 2002, XIII Congreso de Microbiologia de los Alimentos, Université de Rioja). Deux souches de chacune des espèces *Oenococcus oeni* et *Lactobacillus plantarum,* sélectionnées parmi la flore indigène, ont été inoculées à $5.10^6$ UFC/ml dans un vin de la région de la Rioja (sud de l'Espagne) après la fermentation alcoolique. Les souches *Oe. oeni* se sont imposées dans le milieu et ont permis un bon déroulement de la FML, tandis que les souches *L. plantarum* ont régressé jusqu'à ne plus être détectables en fin de FML. Ces résultats montrent une fois de plus que quand Lactobacillus est introduit en milieu alcoolisé, il dépérit et n'est pas capable de fermenter l'acide malique en acide lactique.

Il est par ailleurs bien connu que les bactéries lactiques ont une résistance au stress fortement réduite après séchage, lyophilisation ou congélation. Quand elles sont soumises à des conditions défavorables, telles qu'un degré alcoolique élevé, leur taux de survie décroît drastiquement, et leur activité fermentative ne peut démaner qu'après une période d'adaptation au milieu plus ou moins longue. Pour initier la FML, il faut en outre atteindre une concentration de l'ordre de $10^6$ UFC/ml ce qui nécessitera souvent une phase de multiplication cellulaire permettant à la population de se reconstituer.

La présente invention apporte également une réponse à ce problème grâce aux nouvelles souches de bactéries malolactiques sélectionnées appartenant aux genres Lactobacillus et Pediococcus, celles-ci étant capables d'initier et de réaliser la FML lorsqu'elles sont introduites directement à l'état séché, lyophilisé ou congelé dans un vin à pH moyen ou élevé.

Un procédé d'induction de la FML par inoculation directe de ces bactéries malolactiques résistantes à l'alcool est également un objet de la présente invention.

On appellera "vin" un milieu de fermentation tel qu'un moût à base de jus de raisin ou d'autres fruits, dans lequel la quantité d'alcool produit par fermentation alcoolique est d'au moins 5% en volume. Ce vin peut avoir atteint son degré alcoolique maximum si la fermentation alcoolique est terminée. Les teneurs en alcool sont exprimés par le volume d'alcool rapporté au volume total.

On appellera "vin mature" ou "vin à maturité" un vin dans lequel la FML s'est déroulée complètement, c'est à-dire dans lequel la teneur en acide malique est inférieure à 0,2 g/l. Ce vin mature est microbiologiquement stable contrairement au vin non mature.

Pour un vin, un pH moyen sera de l'ordre de 3,5 à 3,6, et un pH élevé pourra aller d'environ 3,6 jusqu'aux pH maximums rencontrés dans les vins, soit environ 4,0, voire plus.

Par "inoculation directe" on entend introduction dans le milieu de fermentation des bactéries lactiques choisies, sans étape préalable d'acclimatation ou d'adaptation au milieu, à une concentration économiquement acceptable, c'est-à-dire comprise entre 106 et 5.107 UFC/ml de milieu. Une simple réhydratation de 20 minutes dans de l'eau à 22°C peut être opérée.

Les propriétés avantageuses des souches sélectionnées selon l'invention se manifestent par leur capacité à entrer en phase fermentative active pratiquement sans temps de latence pour initier la FML à brefs délais, malgré les conditions défavorables du milieu.

Dans la pratique ceci se traduit par le fait que dès les premiers jours après l'inoculation bactérienne, des fractions importantes de l'acide malique présent dans le milieu sont transformées en acide lactique. Une fois le processus de FML initié, les bactéries inoculées conservent leur position prédominant dans le milieu et assurent le bon déroulement de la FML jusqu'à épuisement du substrat, tout en garantissant que le vin mature finalement produit sera exempt de composés indésirables.

Il est important de noter que les souches selon l'invention sont résistantes à l'alcool même dans des conditions induisant habituellement un stress important et conduisant à un dépérissement cellulaire, en particulier lors de l'inoculation sous forme séchée, lyophilisée ou congelée sans étape préalable d'acclimatation.

Ainsi, une souche de bactérie lactique sélectionnée selon l'invention appartenant au genre Lactobacillus ou Pediococcus, a la capacité de réaliser la conversion de l'acide malique en acide lactique de sorte que, lorsqu'elle est introduite à une concentration comprise entre 106 et $5.10^7$ UFC/ml, directement à l'état séché, lyophilisé ou congelé dans un vin ayant un degré d'alcool de 10% ou plus et un pH supérieur ou égal à 3,5,

    i) elle convertit au moins 5%, et de préférence au moins 10%, de l'acide malique en acide lactique en 5 jours après inoculation dudit vin, et
    ii) - elle convertit au moins 10%, et de préférence au moins 25%, de l'acide malique en acide lactique en 10 jours après inoculation dudit vin.

De manière particulièrement avantageuse une souche de bactérie lactique selon l'invention a une activité fermentative telle que, lorsqu'elle est introduite à une concentration comprise entre 106 et $5.10^7$ UFC/ml, directement à l'état séché, lyophilisé ou congelé dans un vin ayant un degré d'alcool de 10% ou plus et un pH supérieur ou égal à 3,6

    iii) - elle convertit au moins 10%, et de préférence au moins 15%, de l'acide malique en acide lactique en 5 jours après inoculation dudit vin, et
    iv) - elle convertit au moins 25%, et de préférence au moins 40%, de l'acide malique en acide lactique en 10 jours après inoculation dudit vin.

Ces souches sont donc particulièrement intéressantes comme starters de la FML dans des vins de pH moyen ou élevé, en cours ou en fin de fermentation alcoolique. Ce faisant, leur emploi est tout à fait possible dans les conditions usuelles de pH et de concentration alcoolique, leur capacité à réaliser la FML correspondant alors aux performances communément obtenues avec d'autres starters de la FML.

Comme expliqué précédemment, pour s'assurer que des bactéries indigènes ne se développent pas, il peut être intéressant d'inoculer le milieu de fermentation avant la fin de la fermentation alcoolique, sans risquer de produire de l'acidité volatile à partir du sucre encore présent dans le milieu. Selon un caractéristique avantageuse de la présente invention, les souches de bactérie lactique sélectionnées sont homofermentaires.

Il est en outre intéressant que les souches sélectionnées ne soient pas capables de produire des composés toxiques ou conduisant à des modifications organoleptiques désagréables et d'autres effets négatifs sur le vin. C'est pourquoi, l'invention concerne également les souches de bactéries lactiques sélectionnées ne produisant pas d'amines biogènes à partir des précurseurs aminés présents dans le vin, ne dégradant pas le glycérol ni l'acide tartrique. De préférence, les souches selon l'invention possèdent l'ensemble de ces propriétés.

Selon un mode de réalisation avantageux, la présente invention concerne une souche de bactérie lactique possédant l'ensemble des caractéristiques définies précédemment, et ayant la capacité, lorsqu'elle est introduite directement à une concentration de 2.106 UFC/ml, dans un vin à une température supérieure ou égale à 18°C, ayant une teneur en $SO_2$ comprise entre 0 et 15 mg/l, une teneur en alcool supérieure ou égale à 10 %, et un pH de 3,7 ou plus

    i) - de convertir 15 % de l'acide malique en acide lactique en 5 jours après inoculation dudit vin, et
    ii) - de convertir 40 % l'acide malique en acide lactique en 10 jours après inoculation dudit vin.

De manière particulièrement avantageuse, ladite souche de bactérie lactique possède l'ensemble des caractéristiques définies précédemment, et a la capacité, lorsqu'elle est introduite directement à une concentration de $2.10^6$ UFC/ml, dans un vin à une température supérieure ou égale à 18°C, ayant une teneur en $SO_2$ comprise entre 0 et 15 mg/l, une teneur en alcool supérieure ou égale à 10 %, et un pH de 3,7 ou plus

i) - de convertir 50 % de l'acide malique en acide lactique en 5 jours après inoculation dudit vin, et

ii) - de convertir 80 % l'acide malique en acide lactique en 10 jours après inoculation dudit vin.

Les souches possèdent les caractéristiques ci-dessus lorsqu'elles sont introduites dans un vin ayant une teneur en alcool de 10 %, voire de 12 % et même de 13 %. Bien entendu, ces souches présentent des caractéristiques identiques-ou-meilleures lorsqu'elles sont introduites plus précocement dans le vin, par exemple alors que la teneur en alcool n'est encore que de 5%.

Comme indiqué précédemment, les bactéries malolactiques sélectionnées appartiennent de manière inattendue, aux genres Lactobacillus ou Pediococcus, jusque là considérés comme inaptes à se développer en milieu alcoolisé. En particulier elles sont sélectionnées dans le groupe formé de *Lactobacillus plantarum, Lactobacillus casei, Lactobacillus delbrückii, Pediococcus acidilactici, Pediococcus* damnosus *Pediococcus pentosaceus, Pediococcus parvulus, Pediococcus cerevisiae.* damnosus

En particulier, sont revendiquées les souches malolactiques homofermentaires suivantes: *Lactobacillus plantarum* CNCM I-2924, *Pediococcus acidilactici* CNCM MA 18/5M, déposées dans une collection de culture de microorganismes conformément aux dispositions du Traité de Budapest, règle 6.1.

Une préparation de bactéries lactiques destinée à être utilisée comme starter de la FML, c'est à-dire pour l'ensemencement d'un vin en vue de l'induction de la FML, peut comprendre une ou plusieurs souches de bactéries malolactiques telles que définies précédemment, et éventuellement d'autres ingrédients connus de l'homme du métier. De telles préparations peuvent se présenter à l'état liquide, séché, lyophilisé ou congelé.

Les bactéries malolactiques résistantes à l'alcool décrites ci-dessus ou une préparation de celles-ci sont destinées à être mises en oeuvre pour la réalisation contrôlée de la FML dans des vins de pH moyen ou élevé, notamment dans un procédé objet de la présente invention.

Ce procédé de conversion de l'acide malique en acide lactique dans un vin ayant un pH supérieur ou égal à 3,5, consiste à introduire une préparation comprenant au moins une souche de bactérie lactique telle que précédemment décrite, directement à l'état séché, lyophilisé ou congelé dans ledit vin, à une concentration comprise entre 106 et $5.10^7$ UFC/ml, à une température supérieure ou égale à 18°C, lorsque le degré d'alcool a atteint au moins 10% et à maintenir le vin dans des conditions permettant le déroulement de la FML, pour obtenir un vin mature dont la teneur en acide malique est inférieure à 0,2 g/l.

Le procédé peut être appliqué avantageusement à n'importe quel vin dont le pH est supérieur ou égal à 3,5 sans limitation supérieure. Il est mis en oeuvre de manière particulièrement avantageuse pour des vins de pH 3,6 et plus, pour lesquels le risque de développement de bactéries indésirables à croissance rapide est particulièrement élevé.

Si le viticulteur le souhaite, et afin d'éviter un démarrage de la FML par des bactéries indigènes incontrôlées, une préparation de bactéries lactiques selon l'invention peut être introduite dans le vin en cours de fermentation, par exemple lorsque le degré d'alcool a atteint 5 % ou davantage, c'est-à-dire alors que la quantité de sucre présente dans le vin est encore importante. Dans ce cas, les bactéries lactiques entrant dans la préparation d'ensemencement sont toutes homofermentaires, de manière à éliminer le risque de production d'acidité volatile.

En outre, le procédé de conversion de l'acide malique en acide lactique objet de la présente invention, est mis en oeuvre de préférence à l'aide d'une préparation de bactéries lactiques possédant une ou plusieurs des caractéristiques suivantes (l'idéal étant qu'elles les possèdent toutes à la fois) :

- elles ne produisent pas d'amine biogène à partir des précurseurs aminés
- elles ne dégradent pas le glycérol,
- elles ne dégradent pas l'acide tartrique.

Les préparations bactériennes employées dans le procédé revendiqué sont préparées à partir d'une ou plusieurs souches appartenant aux genres Lactobacillus ou Pediococcus, et peuvent être choisies parmi les espèces *Lactobacillus plantarum, Lactobacillus casei, Lactobacillus delbrückii, Pediococcus acidilactici, Pediococcus* damnosus, *Pediococcus pentosaceus, Pediococcus parvulus* ou *Pediococcus cerevisiae.*

En particulier, peuvent être utilisées une ou plusieurs souches de bactérie malolactique sélectionnée dans le groupe composé *de Lactobacillus plantarum* CNCM I-2924 et *Pediococcus acidilactici* CNCM MA 18/5M.

Par exemple, on pourra ajouter à un vin de pH supérieur ou égal à 3,7 dont la concentration en alcool est supérieure à 11%, une préparation de bactéries malolactiques comprenant *Lactobacillus plantarum* CNCM I-2924.

On pourra aussi inoculer un vin de pH supérieur ou égal à 3,7 dont la concentration en alcool est supérieure à 12% avec une préparation de bactéries malolactiques comprenant *Pediococcus acidilactici* CNCM MA 18/5M.

Des concentrations économiquement acceptables sont de l'ordre de $5.10^5$ à $5.10^7$ UFC/ml vin. De préférence on inocule le vin avec $2.10^6$ UFC/ml. Les bactéries lactiques ou les préparations de bactéries lactiques sont introduites directement dans le milieu de fermentation sans étape préalable d'acclimatation. Lorsque les bactéries sont ajoutées dans le vin sous forme lyophilisée, elles subissent une simple réhydratation pendant une vingtaine de minutes.

La présente invention peut être utilisée pour produire un vin mature obtenu à l'aide d'une préparation de bactéries lactiques telles que décrites précédemment

Les exemples suivants permettront d'illustrer plus en détail la réalisation de l'invention et les résultats obtenus. Il est à noter que les modes de réalisation incluant la souche lactoloacillus plantarum DSM 9916 ne sont sont fournis qu'à tire illustratif.

## EXEMPLE 1

### Protocole de sélection des souches

Un screening de souches naturelles de bactéries lactiques issues de vins fermentés ou de jus de fruits fermentés a été réalisé. Des isolats de bactéries lactiques naturelles ont été soumis à des pressions de sélection selon les critères suivants:

- Résistance à des taux l'alcool $\geq$ 10%
- Croissance à basse température (15°C)
- Pas de formation d'amines biogènes
- Pas de dégradation du glycérol
- Pas de dégradation de l'acide tartrique

## EXEMPLE 2

### Détermination de la résistance à l'éthanol

Les bactéries malolactiques sont cultivées dans un bouillon CMB. Le pH du milieu est ajusté à 3,5 avec de la soude 6N. Les tubes à essais sont remplis avec 5 ml de milieu CMB et ensuite autoclavés pendant 15 minutes à 121°C. Après refroidissement, les niveaux d'alcool sont ajustés avec de l'éthanol pur à : 4%, 6%, 8%, 10%, 12% et 14%. Les tubes sont inoculés à 0,5%, d'une culture MRS cultivée pendant 48h (Garvie, 1967, J. gen. Microbiol, vol. 48, pp. 431-4.38). La croissance est suivie par mesure de la turbidité à 600 nm après 3 semaines.

## EXEMPLE 3

### Détermination de la croissance à basse température

La croissance à basse température est testée dans le même milieu selon le même protocole que celui utilisé pour la détermination de la résistance à l'alcool décrit à l'exemple 1. La croissance est suivie par mesure de la turbidité à 600 nm après 3 semaines.

## EXEMPLE 4

### Screening de production d'amines biogènes à partir d'acides aminés

La sélection de souches ne produisant pas l'amines biogènes a été réalisée par culture sur un milieu modèle MECM. Les amines biogènes qui ont été recherchées sont celles que l'on trouve le plus souvent en quantité importante dans les vins: l'histamine, la type, la putrescine et la cadavérine. Pour sélectionner les souches ne produisant pas d'amines biogènes, celles-ci sont cultivées sur un milieu modèle MECM supplémenté en acides aminés précurseurs, à savoir l'histidine pour l'histamine, la tyrosine pour la tyramine, l'omithine pour la putrescine et la lysine pour la cadavérine.

| Composition du milieu modèle | Concentrations (g/l) |
| --- | --- |
| Tryptone | 10.0 |
| Extrait de levure | 4.0 |
| Tween 80 | 1.0 |
| MgSO$_4$, 7 H$_2$O | 0.2 |
| MnSO$_4$, 4 H$_2$O | 0.05 |
| Pantothénate de Calcium | 0.01 |
| Glucose | 5.0 |

(suite)

| Composition du milieu modèle | Concentrations (g/l) |
|---|---|
| Fructose | 5.0 |
| Acide L-malique | 4.0 |

Le pH est ajusté à 5,0 avec HCl ou NaOH et le milieu est autoclavé à 115°C pendant 30 min. Une solution mère d'acides aminés précurseurs à 100 mg/l, stérilisée par filtration, est ajoutée au milieu modèle. La concentration finale du milieu modèle supplémenté est de :

- histidine 10 mg/l
- tyrosine 10 mg/l
- ornithine 10 mg/l
- lysine 10 mg/l

Des pré-cultures des souches de bactéries lactiques sont réalisées dans un milieu MRS, puis les cellules sont lavées dans un tampon phosphate, et remises en suspension dans le même volume de tampon phosphate. Le milieu modèle supplémenté est ensuite inoculé à 1 % avec les souches à tester. Après la réalisation de la FML (dégradation de l'acide malique), les échantillons sont centrifugés et les surnageants sont congelés pour l'analyse des amines biogènes. Seules les bactéries ne formant pas d'amines biogènes à partir des acides aminées précurseurs sont conservées.

**EXEMPLE 5**

**Contrôle du métabolisme du glycérol et de l'acide tartrique**

L'étude de la dégradation de l'acide tartrique et du glycérol est réalisée dans le milieu MECMb, comme suit

| Composition du milieu MECMb | Concentration (g/l) |
|---|---|
| Tryptone | 10.0 |
| Extrait de levure | 4.0 |
| Tween 80 | 1.0 |
| $MgSO_4$, 7 $H_2O$ | 0.2 |
| $MnSO_4$, 4 $H_2O$ | 0.05 |
| Pantothénate de calcium | 0.01 |

Les différents composés suivants sont ajoutés au milieu MECMb pour obtenir différents milieux supplémentés:

| Composé | Concentration (g/l) |
|---|---|
| Glucose | 5.0 |
| Fructose | 5.0 |
| Glycérol | 5.0 |
| TMC: acide tartrique + acide malique + acide citrique: | |
| Acide tartrique | 7.0 |
| Acide malique | 4.0 |
| Acide citrique | 0.4 |

La dégradation est testée dans les différentes combinaisons suivantes:

| Combinaison | Milieu |
|---|---|
| 1 | MECMb |
| 2 | MECMb + TMC |
| 3 | MECMb + TMC + glucose |

(suite)

| Combinaison | Milieu |
|---|---|
| 4 | MECMb + TMC + fructose |
| 5 | MECMb + TMC + glycérol |
| 6 | MECMb + TMC + glucose + fructose + glycérol |

Les bactéries, préalablement cultivées dans du milieu MRS, sont lavées et sont utilisées pour inoculer les milieux MECMb supplémentés à 1%(v:v). Les cultures sont incubées à 28°C pendant 7-15 jours et les métabolites formés sont analysés. Seules les bactéries qui ne dégradent ni l'acide tartrique, ni le glycérol sont conservées.

**EXEMPLE 6**

**Essais de lyophilisation**

Les souches ayant donné une réponse positive à l'ensemble des critères de sélection sont testées en vue d'une production sous la forme lyophilisée. Deux, souches de *Lactobacillus plantarum* et une souche Pediococcus avec les caractéristiques données, ont été produites sous la forme lyophilisée pour l'inoculation directe des vins. La souche *Lactobacillus plantarum* DSM 9916 a été isolée dans un vin Chardonnay californien, la souche *Lactobacillus plantarum* CNCM I-2924 a été isolée à partir d'un moût de fruits destiné être distillé, qui a été conservé par ajout d'acide et de $SO_2$.

**EXEMPLE 7**

**Tolérance à l'alcool**

La tolérance à l'éthanol a été testée dans le milieu CMB pour les souches suivantes:

- les 2 souches sélectionnées de *Lactobacillus plantarum* CNCM I-292A et DSM-9916,
- la souche sélectionnée de *Pediococcus acidilactici* CNCM MA 18/5M,
- une souche commerciale de *Lactobacillus plantarum* (Viniflora) préconisée pour l'inoculation de jus,
- la souche de *Lactobacillus plantarum* CNCM MA 18/5U commercialisée pour la nutrition animale,
- la souche de *Lactobacillus casei* CNCM MA 542/2V, commercialisée pour la nutrition humaine,
- la souche de *Oenococcus oeni* EQ54, commercialisée par Lallemand pour l'ensemencement direct.

Les cellules ont été cultivées sur milieu CMB contenant 0%, 4%, 6%, 8%, 10%, 12 % et 14% d'alcool éthylique. La croissance bactérienne a été déterminée à 21 jours par mesure spectrophotométrique de la densité optique DO à 600 nm.

La tolérance à l'alcool TA(x) des souches en présence d'une quantité d'alcool x%, est représentée par le taux de survie après 21 jours dans en milieu alcoolisé rapporté au taux de survie après 21 jours dans un milieu sans alcool.

$$TA(x) = DO \text{ à } x\% \text{ d'alcool } / DO \text{ à } 0\% \text{ d'alcool}$$

Les résultats sont présentés dans le Tableau 1.

TABLEAU 1

| Souches | Taux de survie (%) aux teneurs en alcool: | | | | | |
|---|---|---|---|---|---|---|
| | 4% | 6% | 8% | 10% | 12% | 14% |
| *L. pl.* CNCM 1-2924 | 90 | 76 | 71 | 66 | 62 | 3 |
| *L. pl.* DSM-9916 | 88 | 84 | 71 | 68 | 57 | 3 |
| *P. acid.* CNCM-MA18/5M | 114 | 106 | 102 | 71 | 46 | 8 |
| *L. pl.* Viniflora | 119 | 96 | 75 | 41 | 6 | 6 |
| *L. pl.* CNCM MA 18/5U | 82 | 68 | 52 | 25 | 6 | 4 |
| *L. cas.* CNCM MA 542/2V | 96 | 134 | 78 | 10 | 7 | 7 |
| *Oe. oeni* EQ54 | 100 | 100 | 95 | 87 | 78 | 77 |

Parmi les souches *Lactobacillus plantarum et Pediococcus acidilactici*, seules les souches sélectionnées de *L. plantarum* DSM-9916 et CNCM I-2924 et la souche *P. acidilactici* CNCM-MA18/SM ont un taux de survie supérieur à 50% après 21 jours dans un milieu contenant 10 % d'alcool, et peuvent se développer à des concentrations en alcool supérieures à 12 %. Ces valeurs sont également atteintes avec la souche *Oe. oeni* EQ54.

## EXEMPLE 8

### Dégradation de l'acide malique par *L. plantarum* CNCM I-2924

La FML a été réalisée à l'aide de la souche *L. plantarum* CNCM I-2924 par inoculation directe sous forme lyophilisée, à raison de $2,0.10^6$ UFC/ml, après réalisation de la fermentation alcoolique dans les vins suivants,:

- Vin test pH = 3,6
- Vin des Vignerons de Buzet, pH = 3,54

Les essais sont réalisés au laboratoire, en flacons de 200 ml.

Préparation du vin test

Le vin test a été fabriqué à partir de jus de raisins commerciaux en trois étapes:
A) Vinification:

1 - Le jus est supplémenté par 70g/l de dextrose.
2 - Le jus est inoculé avec 20g/hl de levure sèche active Lalvin CY3079; la levure est au préalable réhydratée dans un petit volume de jus à 30°C pendant 30 minutes.
3 - La fermentation est conduite à une température comprise entre 20 et 25°C.
4 - Après une semaine, la concentration en sucres résiduels est mesurée. Si la concentrations en sucres résiduels est inférieure à 2g/l, le vin est clarifié.

B) Clarification:

1 - Le vin est clarifié par centrifugation (8500 rpm, 10 mn)
2 - Le vin est placé à 4°C pendant 8 jours.
3 - Les cristaux d'acide tartrique sont éliminés par centrifugation (8500 rpm, 10 mn)

C) Standardisation:
Le vin est analysé après fermentation alcoolique et avant inoculation de préparations lyophilisés de *L. plantarum* DSM-9916, le pH est ajusté à 3,6, la teneur en acide malique est portée à 5 g/l. Le vin test ainsi obtenu a les caractéristiques suivantes:

| | |
|---|---|
| Alcool (%vol) | 11,94 |
| Sucres résiduels (g/l) | 0,0 |
| $SO_2$ libre (mg/l) | 4 |
| $SO_2$ total (mg/l) | 5 |
| Acide acétique (g/l) | 0,23 |
| pH | 3,18 ajusté à 3,6 |
| Acidité totale (g/l $H_2SO_4$) | 5,0 |
| Acide malique (g/l) | 3,1 porté à 5,0 |

Vin rouge (Vignerons de Buzet)

Le vin rouge utilisé est un vin commercial produit par les Vignerons de Buzet. Le vin a été seulement filtré et conservé à 4°C.
Le vin a été analysé après fermentation alcoolique et avant inoculation de préparations lyophilisées de *L.*

*plantarum* DSM-9916.

| | |
|---|---|
| Alcool (%vol) | 12,30 |
| Sucres résiduels (g/l) | 0,5 |
| $SO_2$ libre (mg/l) | 4 |
| $SO_2$ total (mg/l) | 5 |
| Acide acétique (g/l) | 0,17 |
| pH | 3,54 |
| Acidité totale (g/l $H_2SO_4$) | 3,6 |
| Acide malique (g/l) | 1,5 |

La préparation bactérienne lyophilisée est réhydraté dans de l'eau à 22°C pendant 20 minutes. La solution est ensuite ajoutée directement dans le vin après fermentation alcoolique. La survie dans le vin et la dégradation de l'acide malique sont mesurées jusqu'à épuisement du substrat Pour chaque vin, deux essais ont été réalisés. Un lot non ensemencé a servi de témoin.

Taux de survie

Le tableau 2 donne les résultats des taux survie de *L. plantarum* CNCM I-2924, 2 jours et 14 jours après inoculation.

TABLEAU 2

| Vin | Inoculation (UFC/ml) | Population à Jo (UFC/ml) | Population à J2 (UFC/ml) | % survie à J2 | Population à J14 (UFC/ml) | % survie à J14 |
|---|---|---|---|---|---|---|
| Test | 2,0E6 | 2,1E6 | 1,9E6 | 90 | 1,8E6 | 86 |
| Test | 2,0E6 | 2,1E6 | 1,85E6 | 89 | 1,65E6 | 79 |
| Buzet | 2,0E6 | 1,9E6 | 1,6E6 | 84 | 1,7E6 | 89 |
| Buzet | 2,0E6 | 1,95E6 | 1,7E6 | 87 | 1,6E6 | 82 |

Dégradation de l'acide malique

Le dosage de l'acide malique est réalisé pour chaque essai à intervalles réguliers à l'aide d'un kit de dosage (kit E 0139 068, Boehringer Mannheim, Allemagne).

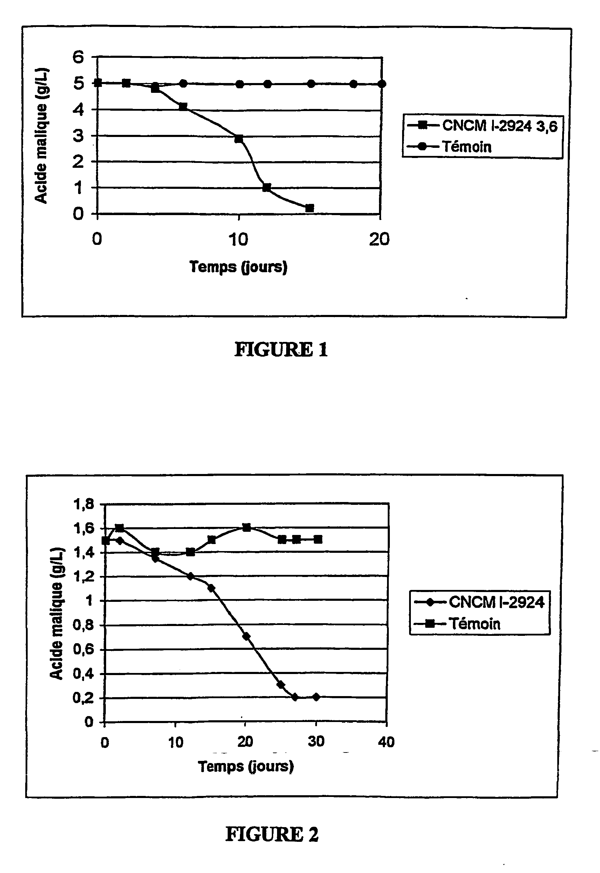

La Figure 1 montre la cinétique de dégradation de l'acide malique dans le vin test à pH=3,6 après inoculation d'une préparation lyophilisée de *L. plantarum* CNCM I-2924.

La Figure 2 montre la cinétique de dégradation de l'acide malique dans le vin de Buzet après inoculation avec une préparation lyophilisée de *plantarum* CNCM I-2924.

**EXEMPLE 9**

**Dégradation de l'acide malique par *L. plantarum* DSM-9916**

La FML a été réalisée à l'aide de la souche *L. plantarum* DSM-9916 par inoculation directe sous forme lyophilisée, à raison de $3,0.10^6$ UFC/ml, après réalisation de la fermentation alcoolique dans le même vin rouge des Vignerons de Buzet, à pH = 3,54, que celui utilisé dans l'exemple précédent Le vin de Buzet a été préparé comme précédemment et possède les mêmes caractéristiques chimiques (voir exemple 8). L'essai a été mené selon le même protocole.

Taux de survie : Le tableau 3 donne les résultats des taux survie de *L. plantarum* CNCM I-2924 , 2 jours et 14 jours après inoculation.

TABLEAU 3

| Vin | Inoculation (UFC/ml) | Population à Jo (UFC/ml) | Population à J2 (UFC/ml) | % survie à J2 | Population à J14 (UFC/ml) | % survie à J14 |
|---|---|---|---|---|---|---|
| Buzet | 3,0E6 | 2,3E6 | 2,3E6 | 97 | 1,8E6 | 78 |
| Buzet | 3,0E6 | 3,1E6 | 2,1E6 | 68 | 1,8E6 | 58 |

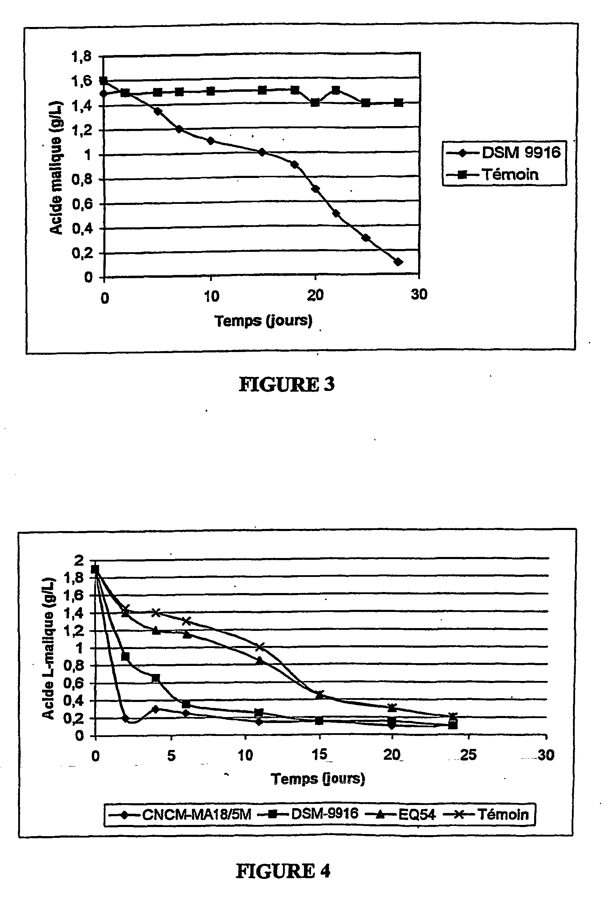

<u>Dégradation de l'acide malique</u> : Le dosage de l'acide malique est réalisé pour chaque essai à intervalles réguliers à l'aide d'un kit de dosage (kit E 0139 068, Boehringer Mannheim, Allemagne). La figure 3 montre la cinétique de dégradation de l'acide malique dans le vin de Buzet après inoculation d'une préparation lyophilisée de *L. plantarum* DSM-9916.

## EXEMPLE 10

**Dégradation de l'acide malique dans un vin Cabernet par *L. plantarum* DSM 9916 et *P. acidilactici* CNCM MA-18/5M - Comparaison avec *Oe. oeni* EQ54.**

La FML a été réalisée dans un vin Cabernet Sauvignon du Chili en 2002, de pH 3,7. Les essais ont été menés dans 4 barriques de 2251 :

- vin ensemencé à l'aide de la souche *L. plantarum* DSM 9916,
- vin ensemencé à l'aide de la souche *P. acidilactici* CNCM MA-18/5M,
- vin ensemencé à l'aide le la souche *OE. oeni* EQ54,
- vin témoin, non ensemencé.

Le vin a été analysé après fermentation alcoolique et avant inoculation de préparations lyophilisées de bactéries lactiques.

| | |
|---|---|
| Alcool (%vol) | 12,9 |
| Sucres résiduels (g/l) | 0,3 |
| $SO_2$ libre (mg/l) | 6 |
| $SO_2$ total (mg/l) | 13 |
| pH | 3,7 |
| Acide malique (g/l) | 1,90 |
| Acide lactique (g/l) | 0,08 |

Les souches ont été inoculées sous forme lyophilisée à raison de $2,0.10^6$ UFC/ml, à 18°C, directement dans le vin après réalisation de la fermentation alcoolique. Le dosage de l'acide malique a été réalisé pour chaque essai à intervalles réguliers à l'aide d'un kit de dosage (kit E 0139 068, Boehringer Mannheim, Allemagne). Les résultats sont présentés sur la Figure 4.

Les cinétiques de dégradation de l'acide malique indiquent une bonne implantation des souches DSM 9916 et CNCM MA-18/5M et un démarrage précoce et efficace de l'activité fermentative. L'essai témoin montre que dans ce vin à pH relativement élevé (pH=3,7), la FML est déclenchée spontanément dès les premiers jours. Dans l'essai réalisé avec ensemencement par *Oe. oeni*, la cinétique de dégradation de l'acide malique est très proche de celle de l'essai témoin, ce qui signifie que des souches indigènes indésirables se sont développées assez rapidement pour supplanter *Oe. oeni*. Le contrôle de la FML garantissant la sécurité fermentaire n'a été obtenu qu'avec les souches *L. plantarum* DSM 9916 et *P. acidilactici* CNCM MA-18/5M.

## EXEMPLE 11

**Dégradation de l'acide malique dans un vin Tempranillo par *L. plantarum* CNCM I-2924 - Comparaison avec *Oe. oeni* EQ54.**

La FML a été réalisée dans un vin Tempranillo de Rioja (Espagne) en 2002, de pH 3,9. Dans les vins de cette région, on assiste souvent au déclenchement spontané de la FML qui se traduit généralement par des taux élevés d'amines biogènes. Les essais ont été menés dans 3 barriques de 225 1.

- vin ensemencé à l'aide de la souche *L. plantarum* CNCM I-2924,
- vin ensemencé à l'aide le la souche *Oe. oeni* EQ54,
- vin témoin, non ensemencé.

Le vin a été analysé après fermentation alcoolique et avant inoculation de préparations lyophilisées de bactéries lactiques.

| | |
|---|---|
| Alcool (%vol) | 11,98 |
| Sucres résiduels (g/l) | 0,2 |
| $SO_2$ libre (mg/l) | 4 |
| $SO_2$ total (mg/l) | 11 |
| pH | 3,9 |
| Acide malique (g/l) | 2,35 |
| Acide lactique (g/l) | 0,05 |

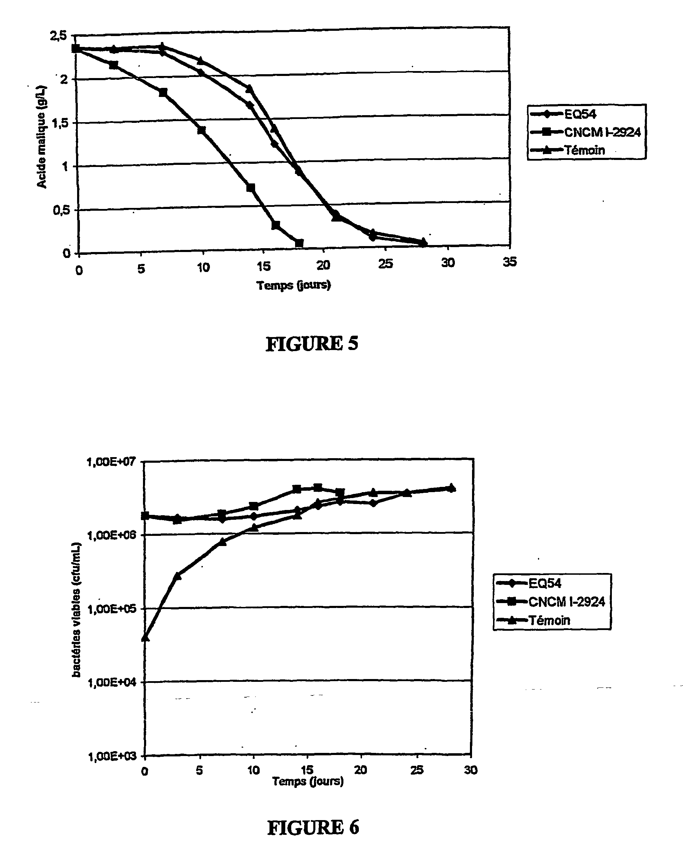

Les souches ont été inoculées directement sous forme lyophilisée à raison de 2,0.106 UFC/ml, à 18°C, après réalisation de la fermentation alcoolique. Le dosage de l'acide malique est réalisé pour chaque essai à intervalles réguliers à l'aide d'un kit de dosage (kit E 0139 068, Boehringer Mannheim, Allennagne). En parallèle, la croissance bactérienne a été mesurée dans les trois cuves. Les résultats sont présentés sur la Figures 5 et 6.

La cinétique de dégradation de l'acide malique (Figure 5) montre une induction rapide de la fermentation avec *L. plantarum* CNCM I-2924. En revanche, la FML ne démarre qu'après 10 jours dans la cuve ensemencée avec *Oe. oeni* EQ54 et dans la cuve témoin: on n'observe pas de différence entre ces deux essais, ce qui signifie que des souches indigènes indésirables se sont développées assez rapidement pour supplanter *Oe. oeni.*

Le suivi de la croissance cellulaire (Figure 6) montre par ailleurs que la population d'*Oe. oeni* EQ54 n'a pas chuté lors de l'inoculation dans le vin et est restée au même niveau que la population de *L. plantarum.* On peut donc attribuer le retard de démarrage de la FML par *Oe. oeni* malgré une concentration cellulaire à priori satisfaisante, à une incapacité à induire une activité fermentative dans les conditions de forte concentration alcoolique et pH élevé. Le taux de survie ne constitue pas dans ce cas le critère décisif pour assurer un contrôle efficace de la FML.

## Revendications

1. Souche de bactérie lactique sélectionnée appartenant au genre Lactobacillus ou Pediococcus, ayant la capacité de réaliser la conversion de l'acide malique en acide lactique, ladite bactérie étant *caractérisée en ce que* lorsqu'elle est introduite à une concentration comprise entre $10^6$ et $5.10^7$ UFC/ml, directement à l'état séché, lyophilisé ou congelé dans un vin ayant un degré d'alcool de 10% ou plus et un pH supérieur ou égal à 3,5,

   i) - elle convertit au moins 5%, et de préférence au moins 10%, de l'acide malique en acide lactique en 5 jours après inoculation dudit vin, et

   ii) - elle convertit au moins 10%, et de préférence au moins 25%, de l'acide malique en acide lactique en 10 jours après inoculation dudit vin.

2. Souche de bactérie lactique selon la revendication 1, *caractérisée en ce que*, lorsqu'elle est introduite à une concentration comprise entre $10^6$ et $5.10^7$ UFC/ml, directement à l'état séché, lyophilisé ou congelé dans un vin ayant un degré d'alcool de 10% ou plus et un pH supérieur ou égal à 3,6:

   iii) - elle convertit au moins 10%, et de préférence au moins 15%, de l'acide malique en acide lactique en 5 jours après inoculation dudit vin, et

   iv) - elle convertit au moins 25%, et de préférence au moins 40%, de l'acide malique en acide lactique en 10 jours après inoculation dudit vin.

3. Souche de bactérie lactique selon l'une des revendications précédentes, *caractérisée en ce qu'*elle est homofermentane,

4. Souche de bactérie malolactique selon l'une des revendications précédentes, possédant, en outre une ou plusieurs des caractéristiques suivantes:

   - elle ne produit pas d'amine biogène à partir des précurseurs aminés
   - elle ne dégrade pas le glycérol,
   - elle ne dégrade pas l'acide tartrique.

5. Souche de bactérie lactique selon la revendication 4, *caractérisée en ce qu'*elle a la capacité, lorsqu'elle est introduire directement à une concentration de 2,106 UFC/ml, dans un vin à une température supérieure ou égale à 18°C, ayant une teneur en $SO_2$ comprise entre 0 et 15 mg/l, une teneur en alcool supérieure ou égale à 10 %, et un pH de 3,7 ou plus

    i) - de convertir 15 % de l'acide malique en acide lactique en 5 jours après inoculation dudit vin, et
    ii)- de convertir 40 % l'acide indique en acide lactique en 10 jours après inoculation dudit yin.

**6.** Souche de bactérie lactique selon la revendication 4, *caractérisée en ce* qu'elle a la capacité, lorsqu'elle est introduite directement à une concentration da 2.106 UFC/ml, dans un vin à une température supérieure ou égale à 18°C, ayant une teneur en $SO_2$ comprise entre 0 et 15 mg/l, une teneur en alcool supérieure ou égale à 10 %, et un pH de 3,7 ou plus

    i) - de convertir 50 % de l'acide malique en acide lactique en 5 jours après inoculation dudit vin, et
    ii) - de convertir 80 % l'acide malique en acide lactique en 10 jours après inoculation dudit vin.

**7.** Souche de bactérie malolactique selon l'une des revendications précédentes sélectionnée dans le groupe formé de *Lactobacillus plantararum, Lactobacillus caset, Laclobacillus delbrückil, Pediococcus acidilactici, Pediococcus* damnosus *Pediococcus pentosaceus, Pediococcus parvulus, Pediococcus cervisiae.*

**8.** Souche de bactérie malolactique selon la revendication précédente choisie dans le groupe composé de *Lactobacillus plantarum* CNCM I-2924 on *Pediococcus acidilactici* CNCM MA 18/5M.

**9.** Préparation de bactéries lactiques comprenant une ou plusieurs souches selon l'une des revandications précédentes,

**10.** Procédé de conversion de l'acide malique en acide lactique dans un vin ayant un pH supérieur ou égal à 3,5, consistant à introduire une préparotion de bactéries lactiques selon la revendication 9, directement à l'état séché, lyophilisé ou congelé dans ledit vin, à une concentration comprise entre $10^6$ et $5.10^7$ UFC/ml, à une température supérieure ou égale à . 18°C, lorsque la degré d'alcool a atteint au moins 10 % et à maintenir le vin dans des conditions permettant le déroulement de la FML, pour obtenir un vin mature ayant une teneur en acide malique inférieure à 0,2 g/l.

**11.** procédé conversion de l'acide malique en acide lactique dans un vin ayant un pH supérieur ou égal à 3,5, consistant à introduire une préparation de bactérides lactiques comprenant une ou plusieurs souches selon la revendication 3, directement à l'état séché, lyophilisé ou congelé dans ledit vin, à une concentration comprise entre $10^6$ et $5.10^7$ UFC/ml, à une température supérieure ou égale à 18°C, lorsque le degré d'alcool a atteint au moins 5 %, et à maintenir le vin dans des conditions perméttant le déroulement de la FML, pour obtenir un vin mature ayant une teneur en acide malique inférieure à 0,2 g/l.

**12.** procédé de conversion de l'acida malique en acide lactique selon l'une des revendications 10 ou 11, dans lequel ladite préparation possède en outre une ou plusieurs des caracteristique suivantes:

    - elle ne produit pas d'amine biogène à partir des précurseurs aminés
    - elle no dégrade pas le glycérol,
    - elle ne dégrade pas l'acide tartrique.

**13.** Procède de conversion de l'acide malique en acide lactique selon l'une des revendications 10 à 12, dans lequel ladite préparation comprend une ou plusieurs souches de bactérie malolactique sélectionnée dans le groupe formé de *Lactobacillus plantarum, Lactobacillus casei, Lactobacillus delbrückii, Pediococcus acidilactici, Pediococcus* damnosus*, Pediococcus pentosaceus, Pediococcus parvulus, Pediococcus cerevisiae.*

**14.** Procède de conversion de l'acide malique en acide lactique selon l'une des revendications 11 à 13, dans lequel ladite préparation comprend une ou plusieurs souches de bactérie malolactique sélectionnée dans le groupe composé de *Lactobacillus plantarum* CNCM 1-1924 *Pediococcus acidilactici* CNCM MA 18/5M.

**15.** Procédé selon la revandication 14 dans lequel une préparation de bactéries malolactiques comprenant *Lactobacillus plantarum* CNCM I-2924 est ajoutée à un vin de pH supérieur ou égal à 3,9 dont la concentration en alcool est supérieure à 11%,

**16.** Procédé selon la revendication 14 dans lequel une préparation de bactéries malolactiques comprenant *Pediococcus acidilactici.* CNCM MA-18/5M est ajoutée à un vin de pH supérieur ou égal à 3,7 dont la concentration en alcool est supérieure à 12%.

**Patentansprüche**

**1.** Ausgewählter Milchsäurebakterienstamm, der zur Gattung *Lactobacillus* oder *Pediococcus* gehört und Äpfelsäure zu Milchsäure umsetzen kann, wobei das Bakterium **dadurch gekennzeichnet ist, dass** es, wenn es in einer Konzentration zwischen $10^6$ und $5.10^7$ kbE/ml direkt im trockenen Zustand, lyophilisiert oder gefroren in einen Wein mit einem Alkoholgehalt von 10% oder mehr und einem pH-Wert von größer oder gleich 3,5 eingeführt wird:

  i) - innerhalb von 5 Tagen nach der Impfung des Weins wenigstens 5% und vorzugsweise wenigstens 10% der Äpfelsäure zu Milchsäure umsetzt; und
  ii) - innerhalb von 10 Tagen nach der Impfung des Weins wenigstens 10% und vorzugsweise wenigstens 25% der Äpfelsäure zu Milchsäure umsetzt.

**2.** Milchsäurebakterienstamm gemäß Anspruch 1, **dadurch gekennzeichnet, dass** er, wenn er in einer Konzentration zwischen $10^6$ und $5 \cdot 10^7$ kbE/ml direkt im trockenen Zustand, lyophilisiert oder gefroren in einen Wein mit einem Alkoholgehalt von 10% oder mehr und einem pH-Wert von größer oder gleich 3,6 eingeführt wird:

  iii) - innerhalb von 5 Tagen nach der Impfung des Weins wenigstens 10% und vorzugsweise wenigstens 15% der Äpfelsäure zu Milchsäure umsetzt; und
  iv) - innerhalb von 10 Tagen nach der Impfung des Weins wenigstens 25% und vorzugsweise wenigstens 40% der Äpfelsäure zu Milchsäure umsetzt.

**3.** Milchsäurebakterienstamm gemäß einem der vorstehenden Ansprüche, **dadurch gekennzeichnet, dass** er homofermentativ ist.

**4.** Malolaktischer Bakterienstamm gemäß einem der vorstehenden Ansprüche, der weiterhin eines oder mehrere der folgenden Merkmale besitzt:

  - er produziert ausgehend von Aminvorstufen kein biogenes Amin;
  - er baut kein Glycerin ab;
  - er baut keine Weinsäure ab.

**5.** Milchsäurebakterienstamm gemäß Anspruch 4, **dadurch gekennzeichnet, dass** er, wenn er direkt in einer Konzentration von $2 \cdot 10^6$ kbE/ml in einen Wein mit einer Temperatur von größer oder gleich 18 °C mit einem $SO_2$-Gehalt zwischen 0 und 15 mg/l, einem Alkoholgehalt von größer oder gleich 10% und einem pH-Wert von 3,7 oder mehr eingeführt wird:

  i) - innerhalb von 5 Tagen nach der Impfung des Weins 15% der Äpfelsäure zu Milchsäure umsetzt; und
  ii) - innerhalb von 10 Tagen nach der Impfung des Weins 40% der Äpfelsäure zu Milchsäure umsetzt.

**6.** Milchsäurebakterienstamm gemäß Anspruch 4, **dadurch gekennzeichnet, dass** er, wenn er direkt in einer Konzentration von $2.10^6$ kbE/ml in einen Wein mit einer Temperatur von größer oder gleich 18 °C mit einem $SO_2$-Gehalt zwischen 0 und 15 mg/l, einem Alkoholgehalt von größer oder gleich 10% und einem pH-Wert von 3,7 oder mehr eingeführt wird:

  i) - innerhalb von 5 Tagen nach der Impfung des Weins 50% der Äpfelsäure zu Milchsäure umsetzt; und
  ii) - innerhalb von 10 Tagen nach der Impfung des Weins 80% der Äpfelsäure zu Milchsäure umsetzt.

**7.** Malolaktischer Bakterienstamm gemäß einem der vorstehenden Ansprüche, der aus der Gruppe ausgewählt ist, die aus *Lactobacillus plantarum, Lactobacillus casei, Lactobacillus delbrueckii, Pediococcus acidilactici, Pediococcus damnosus, Pediococcus pentosaceus, Pediococcus parvulus, Pediococcus cerevisiae* besteht.

**8.** Malolaktischer Bakterienstamm gemäß dem vorstehenden Anspruch, der aus der Gruppe ausgewählt ist, die aus *Lactobacillus plantarum* CNCM I-2924 oder *Pediococcus acidilactici* CNCM MA 18/5M besteht.

**9.** Milchsäurebakterienpräparat, das einen oder mehrere Stämme gemäß einem der vorstehenden Ansprüche umfasst.

**10.** Verfahren zur Umsetzung von Äpfelsäure zu Milchsäure in einem Wein mit einem pH-Wert von größer oder gleich 3,5, umfassend das Einführen eines Milchsäurebakterienpräparats gemäß Anspruch 9 direkt im trockenen Zustand, lyophilisiert oder gefroren in den Wein in einer Konzentration zwischen $10^6$ und $5.10^7$ kbE/ml bei einer Temperatur von größer oder gleich 18°C, wenn der Alkoholgehalt wenigstens 10% erreicht hat, und das Halten des Weines unter Bedingungen, die die Entwicklung der malolaktischen Gärung ermöglichen, um einen reifen Wein mit einem Äpfelsäuregehalt von weniger als 0,2 g/l zu erhalten.

**11.** Verfahren zur Umsetzung von Äpfelsäure zu Milchsäure in einem Wein mit einem pH-Wert von größer oder gleich 3,5, umfassend das Einführen eines Milchsäurebakterienpräparats, das einen oder mehrere Stämme gemäß Anspruch 3 umfasst, direkt im trockenen Zustand, lyophilisiert oder gefroren in den Wein in einer Konzentration zwischen $10^6$ und $5.10^7$ kbE/ml bei einer Temperatur von größer oder gleich 18 °C, wenn der Alkoholgehalt wenigstens 5% erreicht hat, und das Halten des Weines unter Bedingungen, die die Entwicklung der malolaktischen Gärung ermöglichen, um einen reifen Wein mit einem Äpfelsäuregehalt von weniger als 0,2 g/l zu erhalten.

**12.** Verfahren zur Umsetzung von Äpfelsäure zu Milchsäure gemäß einem der Ansprüche 10 oder 11, wobei das Präparat weiterhin eines oder mehrere der folgenden Merkmale besitzt:

- es produziert ausgehend von Aminvorstufen kein biogenes Amin;
- es baut kein Glycerin ab;
- es baut keine Weinsäure ab.

**13.** Verfahren zur Umsetzung von Äpfelsäure zu Milchsäure gemäß einem der Ansprüche 10 bis 12, wobei das Präparat einen oder mehrere malolaktische Bakterienstämme umfasst, die aus der Gruppe ausgewählt sind, die aus *Lactobacillus plantarum, Lactobacillus casei, Lactobacillus delbrueckii, Pediococcus acidilactici, Pediococcus damnosus, Pediococcus pentosaceus, Pediococcus parvulus, Pediococcus cerevisiae* besteht.

**14.** Verfahren zur Umsetzung von Äpfelsäure zu Milchsäure gemäß einem der Ansprüche 11 bis 13, wobei das Präparat einen oder mehrere malolaktische Bakterienstämme umfasst, die aus der Gruppe ausgewählt sind, die aus *Lactobacillus plantarum* CNCM 1-2924, *Pediococcus acidilactici* CNCM MA 18/5M besteht.

**15.** Verfahren gemäß Anspruch 14, wobei ein Präparat von malolaktischen Bakterion, das *Lactobacillus plantarum* CNCM I-2924 umfasst, einem Wein mit einem pH-Wert von größer oder gleich 3,9, dessen Alkoholkonzentration über 11% liegt, zugefügt wird.

**16.** Verfahren gemäß Anspruch 14, wobei ein Präparat von malolaktischen Bakterion, das *Pediococcus acidilactici* CNCM MA 18/5M umfasst, einem Wein mit einem pH-Wert von größer oder gleich 3,7, dessen Alkoholkonzentration über 12% liegt, zugefügt wird.

**Claims**

**1.** Strain of lactic bacterium selected belonging to the *Lactobacillus or Pediococcus genus,* having the capacity to carry out the conversion of malic acid to lactic acid, said bacterium being **characterized in that** when it is introduced at a concentration comprised between $10^6$ and $5.10^7$ CFU / ml, directly, in the dried, lyophilized or frozen form, into a wine having an alcohol content of 10% or more and a pH greater than or equal to 3.5,

i) - it converts at least 5%, and preferably at least 10% of the malic acid into lactic acid in 5 days after inoculation of said wine, and
ii) - it converts at least 10% and preferably at least 25% of the malic acid into lactic acid in 10 days after inoculation of said wine.

**2.** Strain of lactic bacterium according to claim 1, **characterized in that**, when it is introduced at a concentration comprised between $10^6$ and $5.10^7$ CFU / ml, directly, in the dried, lyophilized or frozen form, into a wine having an alcohol content of 10% or more and a pH greater than or equal to 3.6:

iii) - it converts at least 10% and preferably at least 15% of the malic acid to lactic acid in 5 days after inoculation of said wine, and

iv) - it converts at least 25% and preferably at least 40% of the malic acid into lactic acid in 10 days after inoculation of said wine.

3. Strain of lactic bacterium according to one of the preceding claims, **characterized in that** it is homofermentative.

4. Strain of malolactic bacterium according to any of the preceding claims, further having one or more of the following characteristics:

- it does not produce biogenic amine from amino precursors
- it does not degrade glycerol,
- it does not degrade tartaric acid.

5. Strain of lactic bacterium according to claim 4, **characterized in that** it has the ability, when introduced directly at a concentration of $2.10^6$ CFU / ml, in a wine at a temperature above or equal to 18°C, with a $SO_2$ content comprised between 0 and 15 mg / l, an alcohol content greater than or equal to 10%, and a pH of 3.7 or more

i) - to convert 15% of the malic acid to lactic acid in 5 days after inoculation of said wine, and
ii) - to convert 40% malic acid to lactic acid in 10 days after inoculation of said wine.

6. Strain of lactic bacterium according to claim 4, **characterized in that** it has the ability, when introduced directly at a concentration of $2.10^6$ CFU / ml, in a wine at a temperature above or equal to 18°C, with a $SO_2$ content comprised between 0 and 15 mg / l, an alcohol content greater than or equal to 10%, and a pH of 3.7 or more

i) - to convert 50% of the malic acid to lactic acid in 5 days after inoculation of said wine, and
ii) - to convert 80% of malic acid to lactic acid in 10 days after inoculation of said wine.

7. Malolactic bacterial strain according to one of the preceding claims selected from the group consisting of *Lactobacillus plantarum, Lactobacillus casei, Lactobacillus delbriickii, Pediococcus acidilactici, Pediococcus damnosus, Pediococcus pentosaceus* or *Pediococcus parvulus, Pediococcus cerevisiae.*

8. Malolactic bacterial strain according to the preceding claim selected from the group consisting of Lactobacillus plantarum DSM-9916, CNCM I-2924, or *Pediococcus acidilactici* CNCM MA 18/5M.

9. Preparation of lactic acid bacteria comprising one or more strains of one of the preceding claims.

10. A method of converting malic acid to lactic acid in a wine having a pH greater than or equal to 3.5, consisting in introducing a preparation of lactic bacteria according to claim 9, directly, in the dried, lyophilized or frozen form, into said wine, at a concentration comprised between $10^6$ and $5.10^7$ CFU / ml, at a temperature above or equal to 18°C, when the alcohol has reached at least 10% and to hold the wine under conditions permitting the progress of the FML, to obtain a wine with mature a content of malic acid of less than 0.2 g / l.

11. A method of converting malic acid to lactic acid in wine having a pH greater than or equal to 3.5, consisting in introducing a preparation of lactic acid bacteria comprising one or more strains claim 3, according to directly in the dried, lyophilized or frozen form , into said wine at a concentration comprised between $10^6$ and $5.10^7$ CFU / ml, at a temperature above or equal to 18°C, when the alcohol has reached at least 5%, and to hold the wine under conditions enabling the course of the FML, to obtain a mature wine having a content of malic acid of less than 0.2 g / l.

12. A method of converting malic acid to lactic acid according to one of claim 10 or 11, wherein said preparation further has one or more of the following characteristics:

- it does not produce biogenic amine from amino precursors
- it does not degrade glycerol
- it does not degrade tartaric acid.

13. A method of converting malic acid to lactic acid according to one of Claims 10 to 12, wherein said preparation comprises one or more strains of malolactic bacteria selected from the group consisting of *Lactobacillus plantarum, Lactobacillus casei, Lactobacillus delbrickii, Pediococcus acidilactici, Pediococcus damnosus, Pediococcus pen-*

*tosaceus, Pediococcusparvulus, Pediococcus cerevisiae.*

**14.** A method of converting malic acid to lactic acid according to one of claims 11 to 13, wherein said preparation comprises one or more strains of malolactic bacteria selected from the group consisting of Lactobacillus plantarum, CNCM I-2924, *Pediococcus acidilactici* CNCM MA 18/5M.

**15.** The method according to claim 14, wherein a preparation of malolactic bacteria comprising *Lactobacillus plantarum* CNCM I-2924 is added to a wine with a pH greater than or equal to 3.9, whose alcohol concentration is above 11 %.

**16.** The method of claim 14, wherein a preparation comprising malolactic bacteria *Pediococcus acidilactici* CNCM MA 18/5M, is added to a wine with a pH greater than or equal to 3.7 whose alcohol concentration is above 12%.

**FIGURE 1**

**FIGURE 2**

**FIGURE 3**

**FIGURE 4**

**FIGURE 5**

**FIGURE 6**

## RÉFÉRENCES CITÉES DANS LA DESCRIPTION

**Documents brevets cités dans la description**

- EP 0398957 A
- EP 635050 A

**Littérature non-brevet citée dans la description**

- **KUNKEE.** *Adv. Appl. Microbiol.,* 1967, vol. 9, 235-279
- **CARBO et al.** *Riv. Vitic. Envl,* 1995, vol. 48 (4), 29. 38
- **CARRIE et al.** *Revue des Oenoloques,* 2002, vol. 103, 16-18
- **PILONE.** *Wine Industrie Journal,* 1995, vol. 10 (2), 169-173
- **LIN et al.** *Am. J. Envl. Vitric,* 1995, vol. 46 (2), 166-174
- **JOYEUX et al.** *Connaissance Vigne Vin,* 1985, vol. 19 (3), 149-159
- **RIBERAU-GAYON et al.** *Sciences et techniques du vin,* 1975, vol. III
- **LAFON-FOURCADE et al.** *Conn. Vigne Vin,* 1993, vol. 17, 55-71
- **GARVIE.** *J. gen. Microbiol,* 1967, vol. 48, 431-4.38